Europäisches Patentamt

(19) European Patent Office

Office européen des brevets

(11) EP 0 823 202 A1

(12) EUROPEAN PATENT APPLICATION
published in accordance with Art. 158(3) EPC

(43) Date of publication:
11.02.1998 Bulletin 1998/07

(51) Int. Cl.$^6$: **A01G 7/00**, A01G 1/00,
A01N 63/00

(21) Application number: 97905402.0

(22) Date of filing: 26.02.1997

(86) International application number:
PCT/JP97/00551

(87) International publication number:
WO 97/31521 (04.09.1997 Gazette 1997/38)

(84) Designated Contracting States:
AT BE CH DE DK ES FI FR GB GR IE IT LI LU MC
NL PT SE

(30) Priority: 29.02.1996 JP 67432/96

(71) Applicant:
IDEMITSU KOSAN COMPANY LIMITED
Tokyo 100 (JP)

(72) Inventors:
• KOMADA, Hajimu
Age-gun, Mie 514-22 (JP)
• MOCHIZUKI, Masami,
Idemitsu Kosan Company Ltd.
Sodegaura-shi, Chiba 299-02 (JP)

(74) Representative:
Türk, Gille, Hrabal, Leifert
Brucknerstrasse 20
40593 Düsseldorf (DE)

(54) **PLANT CULTIVATING ROCK WOOL, METHOD FOR PRODUCING THE SAME, AND METHOD FOR CULTIVATING PLANTS USING THE ROCK WOOL**

(57) The invention is to provide rock wool for growing plants, with which it is possible to prevent soil-borne diseases with no use of synthetic fungicides and which has no problem in its safety, to provide a method for producing such rock wool, and to provide a method for growing plants using the rock wool.

The invention of claim 1 is to provide rock wool for growing plants, which contains spore cells or mycelial cells of non-pathogenic microorganisms; that of claim 4 is to provide a method for producing rock wool for growing plants of claim 1, which comprises inoculating rock wool with a suspension of spore cells or mycelial cells of non-pathogenic microorganisms, followed by drying the rock wool; and that of claim 9 is to provide a method for growing plants using rock wool, which comprises wetting a rock wool for growing plants of claim 1, followed by growing plants on the rock wool.

FIG.3

■—■ : Treated with F13
      (test group of the invention)
●—● : Not treated with F13(control group)
▲—▲ : Not Inoculated with Pathogen
      (reference group)

Degree of Disease Severity

Number of Days after the Transplantation

**Description**

TECHNICAL FIELD

The present invention relates to rock wool for growing plants, which contains non-pathogenic microorganisms, to a method for producing the rock wool, and to a method for growing plants using the rock wool; and the invention is favorably utilized in the field of agriculture and horticulture.

BACKGROUND ART

Heretofore, hydroponics using rock wool has been put into practice, and is known suitable for growing vegetable and flowers such as tomatoes, etc.

However, as being generally effected in greenhouses, hydroponics is problematic in that a plant disease, once having occurred in some plants being grown, rapidly spreads from those plants to the others often resulting in complete damage to all plants growing in one greenhouse. Recently, therefore, infectious diseases which are caused by soil-borne pathogens, such as those of the genera Fusarium, Verticillium, Pseudomonas and the like, and which have heretofore been considered problematic in soil culture have produced serious problems in hydroponics:

A method of using synthetic fungicides to prevent such soil-borne diseases, for example, Fusarium crown and root rot disease, is poorly practicable because of the high probability of in-crop accumulation of synthetic fungicide in the crop tissues. In addition, in view of the recent trend toward chemical-free crops in consumers' demand, a method is desired capable of preventing such soil-borne infectious diseases with no use of fungicides.

The invention is to solve the problems in the prior art, and its object is to provide rock wool for growing plants, with which it is possible to prevent soil-borne infectious diseases with no use of synthetic fungicides and which has no problem in its safety, to provide a method for producing such rock wool, and to provide a method for growing plants using the rock wool.

DISCLOSURE OF THE INVENTION

Specifically, the invention of claim 1 is to provide rock wool for growing plants, which contains spore cells or mycelial cells of non-pathogenic microorganisms.

The invention of claim 2 is to provide the rock wool for growing plants of claim 1, in which the non-pathogenic microorganisms are those of the genus Fusarium.

The invention of claim 3 is to provide the rock wool for growing plants of claim 2, in which the non-pathogenic microorganisms of the genus Fusarium are those of Fusarium oxysporum (FERM BP-5825).

The invention of claim 4 is to provide a method for producing rock wool for growing plants of claim 1, which comprises inoculating rock wool with a suspension of spore cells or mycelial cells of non-pathogenic microorganisms, followed by drying the rock wool.

The invention of claim 5 is to provide the method of claim 4, in which the non-pathogenic microorganisms are those of the genus

Fusarium.

The invention of claim 6 is to provide the method of claim 5, in which the non-pathogenic microorganisms of the genus Fusarium are those of Fusarium oxysporum (FERM BP-5825).

The invention of claim 7 is to provide the method of claim 4, in which the rock wool is dried to have a water content of not larger than 15 %.

The invention of claim 8 is to provide the method of claim 6, in which the rock wool is dried to have a water content of not larger than 15 %.

The invention of claim 9 is to provide a method for growing plants using rock wool, which comprises wetting a rock wool for growing plants of claim 1, followed by growing plants on the rock wool.

The invention of claim 10 is to provide a method for growing plants using rock wool, which comprises wetting a rock wool for growing plants of claim 2, followed by growing plants on the rock wool.

BRIEF DESCRIPTION OF THE DRAWINGS

Fig. 1 is a graph showing the time-dependent variation in the population density of the cell in rock wool cubes in Example 1.

Fig. 2 is a graph showing the time-dependent variation in the population density of the cell in rock wool cubes as inoculated with only pathogens (control) in Example 1.

Fig. 3 is a graph showing the degree of severity of Fusarium crown and root rot in tomato disease having occurred in the external parts of tomato plants of a first group as treated with non-pathogenic F13 (test group of the invention), that of a second group not treated with non-pathogenic F13 (control group), and that of a third group not inoculated with pathogen (reference group), all in Example 1.

BEST MODE FOR CARRYING OUT THE INVENTION

Now, the invention is described in detail hereinunder.

The invention of claim 1 is to provide rock wool for growing plants, which contains but cells, spore cells or mycelial cells of non-pathogenic microorganisms.

The non-pathogenic microorganisms for use in the invention include, for example, those of the genus Fusarium as in claim 2, which, however, are not limitative. Any other non-pathogenic, useful microorganisms are applicable to the invention. For example, such non-pathogenic, useful microorganisms include filamentous fungi of the genera Gliocladium and Trichoderma, and non-pathogenic bacteria of the genera Pseudomonas and Bacillus.

The microorganisms of the genus Fusarium for use in the invention are non-pathogenic ones having the ability of inducing resistance to pathogens, and include, for example, those of Fusarium oxysporum as in claim 3.

Those non-pathogenic microorganisms were isolated by the inventors from the soil in a farm field in the Shimane University, and have been subjected to international deposition as a strain F13 (after having been transferred from the original deposition on February 19, 1997) in the National Institute of Bioscience and Human-Technology Agency of Industrial Science and Technology of 1-3, Higashi 1-chome, Tsukuba-shi, Ibaraki-ken, Japan; and have received a deposition number of FERM BP-5825. The original deposition was made in the same National Institute of Bioscience and Human-Technology Agency of Industrial Science and Technology on July 20, 1995, and the deposition number is FERM P-15057.

As using such non-pathogenic microorganisms, the invention involves no problem in their safety. The invention uses the non-pathogenic microorganisms for the purpose of inducing resistance to pathogens causing soil-borne infectious diseases, such as a Fusarium crown and root rot disease in tomato and the like, in hydroponics. In addition, such non-pathogenic microorganisms can be used also for the purpose of inducing resistance to pathogens causing soil-borne diseases, such as a Fusarium crown and root rot disease in tomato and the like, in soil culture.

The invention of claim 2 is to provide the rock wool for growing plants of claim 1, in which the non-pathogenic microorganisms are those of the genus Fusarium. In other words, it provides rock wool for growing plants containing spore cells or mycelial cells of non-pathogenic microorganisms of the genus Fusarium.

The rock wool for growing plants of the invention of claim 1 contains spore cells or mycelial cells of non-pathogenic microorganisms such as those mentioned hereinabove; and that of the invention of claim 2 contains spore cells or mycelial cells of non-pathogenic microorganisms of the genus Fusarium. The production of the rock wool of that type is not specifically defined, but the method of claim 4 of the invention favorably produces it.

Specifically, as in claim 4, rock wool is inoculated with a suspension of spore cells or mycelial cells of non-pathogenic microorganisms, and then dried, thereby efficiently giving the rock wool for growing plants of claim 1 of the invention.

More concretely, as in claim 5, rock wool is inoculated with a suspension of spore cells or mycelial cells of non-pathogenic microorganisms of the genus Fusarium, and then dried, thereby efficiently giving the rock wool for growing plants of claim 2 of the invention.

The amount of the suspension of spore cells or mycelial cells of microorganisms of the genus Fusarium to be inoculated into rock wool is not specifically defined, but may be such that the rock wool can have, after having been inoculated therewith and dried, generally from $10^2$ to $10^{11}$ cfu/cm$^3$ rock wool, preferably from $10^3$ to $10^{10}$ cfu/cm$^3$ • rock wool, of the microorganism spore cells or mycelial cells. In general, however, the cell density in the rock wool is not so much lowered, even after having been dried, as will be mentioned hereinunder. Though depending on the type of rock wool, the dried rock wool may often have the original cell density.

In the invention of claim 4, the rock wool as inoculated with a suspension of spore cells or mycelical cells of non-pathogenic microorganisms, such as those of the genus Fusarium, must be dried.

Regarding the degree of the drying, in general, the water content of the dried rock wool must be not larger than 15 %, preferably not larger than 10 %, as in claims 7 and 8.

The dry condition of the rock wool having a water content of not larger than 15 % is desirable in that the non-pathogenic microorganisms such as those of the genus Fusarium as inoculated there into can form their durable morphology in that dry condition, that the microorganism spore cells or mycelial cells are prevented from growing or sprouting with no growth of any other undesirable microorganisms in that dry condition, and that the rock wool in that dry condition is free from any troubles in transporting and handling it.

The drying method is not specifically defined. In general, the wet rock wool may be dried in air.

The shape of the rock wool for use in the present invention is not specifically defined. For example, the rock wool may be in any form of cubes, mats or granules.

In that manner mentioned hereinabove, prepared is the rock wool for growing plants of claim 1 of the invention containing spore cells or mycelial cells of non-pathogenic microorganisms. (In the same manner as above, also prepared is the rock wool for growing plants of claim 2 comprising cells or spores of microorganisms of the genus <u>Fusarium</u>.)

The invention of claim 9 is characterized in that the dry rock wool for growing plants is wetted and plants are grown on the thus-wetted rock wool.

To wet the dry rock wool, employable is any way of wetting it to be in an ordinary condition suitable for growing plants thereon.

Crop plants of, for example, tomatoes, cucumbers, eggplants or the like are grown on the rock wool in that condition. The plants to be grown are not specifically defined. The condition for the growing plants may be any ordinary one. Employable is any of hydroponics and soil culture to which may be applied any ordinary fertilizer.

After having been grown in that manner, the plants well grow to the intended height with no soil-borne infectious diseases including Fusarium crown and root rot disease in tomato and the like, and yield good fruit.

Now, the present invention is described more concretely by means of Examples.

Production Example 1 (production of rock wool cubes for growing plants):

A predetermined amount of a suspension of cells of non-pathogenic F13 [<u>Fusarium oxysporum</u> (FERM BP-5825)] was poured onto rock wool cubes of three different types (A to C) shown in Table 1 to thereby make the cubes have $10^9$ cells/cm$^3$, and thereafter the cubes were dried in air at a temperature of 25°C $\pm$ 1°C and at a humidity of from 50 to 60 %. The drying was continued for 28 days, and stopped. The time-dependent variation in the water loss in the rock wool cubes is shown in Table 1. The rock wool cubes A (trade name: Nittobo) and the rock wool cubes B (trade name: Air-rich) were both 10 cm × 10 cm × 10 cm in size; while the rock wool cubes C (trade name: Glodan) were 10 cm × 10 cm × 5.5 cm. After having been dried, all rock wool cubes had a water content of 5 %.

On the other hand, counted was the number of the living cells of F13 in the rock wool cubes that had been inoculated with F13 and dried at a temperature of 25°C $\pm$ 1°C and at a humidity of from 50 to 60 %. The number of the living cells existed in the non-dried cubes (on day 0) and that in the dried cubes (from day 28 to day 100) are shown in Table 2.

Table 1

| Type of Rock Wool | Water Loss (ml/rock wool cube) | | | | |
|---|---|---|---|---|---|
| | 0 day | 7 days | 14 days | 21 days | 28 days |
| A (Nittobo) | 840 | 560 | 290 | 80 | 0 |
| B (Air-rich) | 840 | 590 | 260 | 70 | 0 |
| C (Glodan) | 500 | 190 | 15 | 0 | 0 |

Table 2

| Type of Rock Wool | Number of Living Cells of F13 (cfu/cm$^3$ rock wool) | | | | | | | |
|---|---|---|---|---|---|---|---|---|
| | 0 day | 28 days | 35 days | 42 days | 49 days | 56 days | 70 days | 100 days |
| A (Nittobo) | $4.1 \times 10^8$ | $3.1 \times 10^4$ | $1.6 \times 10^5$ | $1.2 \times 10^5$ | $1.3 \times 10^6$ | $1.4 \times 10^5$ | $1.3 \times 10^5$ | $1.2 \times 10^4$ |
| B (Air -rich) | $1.0 \times 10^9$ | $5.5 \times 10^5$ | $1.6 \times 10^6$ | $1.1 \times 10^6$ | $1.8 \times 10^5$ | $1.2 \times 10^5$ | $1.1 \times 10^6$ | $1.0 \times 10^5$ |
| C (Glodan) | $2.0 \times 10^7$ | $2.2 \times 10^5$ | $1.1 \times 10^6$ | $1.6 \times 10^5$ | $1.8 \times 10^5$ | $1.4 \times 10^5$ | $1.2 \times 10^4$ | $1.1 \times 10^5$ |

From the data in Table 1, it is known that water as applied thereto was removed from all rock wool cubes on day 28 so that the resulting cubes were restored to their original dry condition.

From the data in Table 2, it is known that the cells of F13 are still living in the dry cubes (on day 28) and therefore can well sprout in the cubes up to day 100 (that is, for a period of 72 days from on day 28 at which the drying was stopped), if wetted with water, to exhibit their ability to induce resistance to pathogens.

Example 1:

Using the rock wool cubes for growing plants as prepared in Production Example 1, plants were grown in hydroponics.

Briefly, 8 dry rock wool cubes that had' been prepared in Production Example 1 were set at intervals of 20 cm on a rock wool slab (1.8 m × 0.3 m × 0.1 m) provided with a separate water supply, to which was dropwise applied a liquid culture medium of Otsuka Ekihi No. 1 and No. 2. The lowermost temperature in this system was set at not lower than 5 °C. Two series each having 8 plants/slab were prepared in each of different groups tested herein.

Tomato plants (variety: House Momotaro) that had been grown in a glass greenhouse for 15 days were transplanted onto the cubes of a test group. After 13 days, applied to each cube was a liquid that had been prepared by incubating cells of a nit-mutant of Fusarium oxysporum f. sp. radicis-lycopersici (this causes a Fusarium crown and root rot disease in tomato) in a potato sucrose broth medium (PSB) with shaking at 25°C for 6 days, thereby making each cube have $10^5$ cells/$cm^3$.

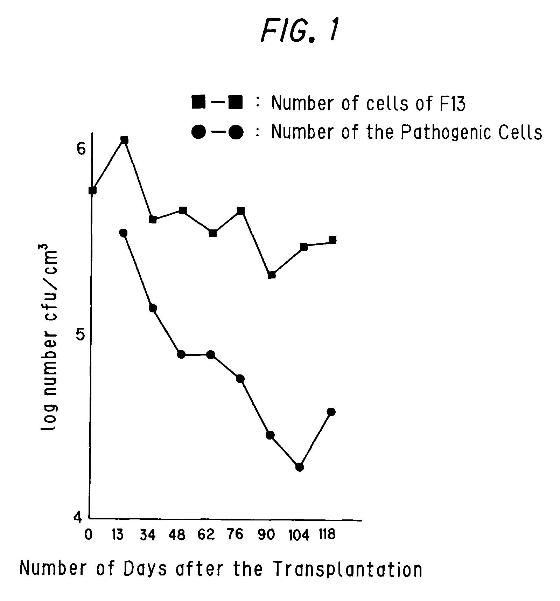

Rock wool was sampled from each cube periodically, and the number of the living cells of the pathogenic and that of the non-pathogenic cells in $1cm^3$ of each rock wool sample were estimated in a modified MMCPA medium and in a Komada' s medium, respectively. The data obtained are plotted in Fig. 1. Fig. 1 shows the time-dependent variation in the cell density in the rock wool cubes tested in Example 1. In Fig. 1, the graph represented by ■ - ■ indicates the number of the non-pathogenic cells of F13, while that represented by ● - ● indicates the number of the living cells of the pathogen.

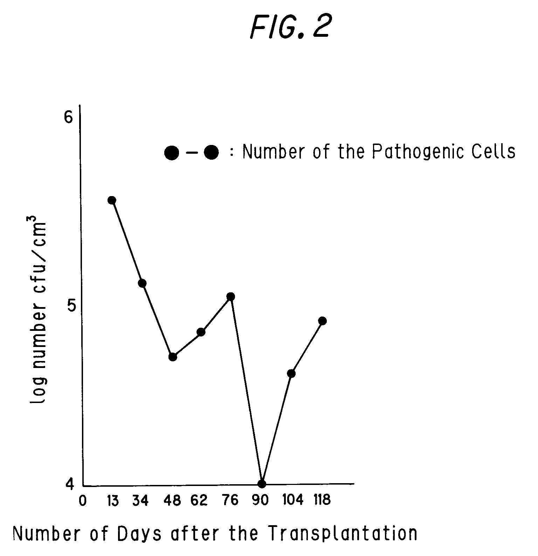

In a control group, only the pathogenic cells were inoculated into the rock wool cubes with no inoculation with the non-pathogenic cells of F13, and tomato plants were grown in the same manner as in the test group of the invention. Fig. 2 is a graph showing the time-dependent variation in the number of the living cells of the pathogenic in rock wool samples in the control group in which the rock wool cubes were inoculated with the pathogenic cells only.

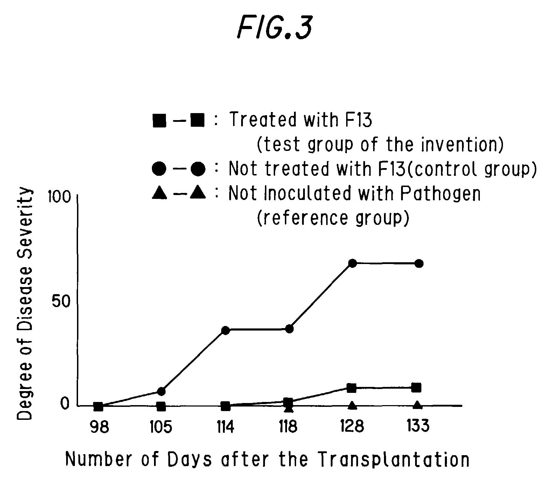

On the other hand, disease incidence of Fusarium crown and root rot in tomato having occurred in the external parts of the tomato plants in each of different groups was measured. The data obtained are plotted in Fig. 3. Fig. 3 is a graph showing the disease incidence of Fusarium crown and root rot in tomato having occurred in the external parts of tomato plants of a first group as treated with non-pathogenic F13 (test group of the invention: ■ - ■), that of a second group not treated with non-pathogenic F13 (control group: ● - ●), and that of a third group not inoculated with pathogen (reference group: ▲ - ▲). The reference group is different from the other groups in that Fusarium crown and root rot pathogens was not applied to the cubes in the former.

The degree of disease incidence in Fig. 3 was obtained according to the following equation.

$$\text{Degree of Disease Incidence} = [\Sigma \text{ (number of plants having the same disease index)} \times \text{(disease index)}] / \{\text{(number of all plants tested)} \times 4\} ] \times 100$$

The disease index is as follows:

0: The plant was not diseased.
1: The plant was partly wilted at its shoot apices.
2: The plant was partly wilted at its leaves.
3: The plant was wholly wilted.
4: The plant was dead.

As in Fig. 1, it is known that the number of cells of the non-pathogenic F13, Fusarium oxysporum (FERM BP-5825), per $cm^3$ of rock wool cubes, which is represented by ■ - ■, was almost on the level of about $10^5$ throughout the test period, while the number of cells of the nit-mutant of the pathogen Fusarium oxysporum f. sp. radicis-lycopersici (this cases a Fusarium crown and root rot disease in tomato), which is represented by ● - ●, was almost on the level of about $10^4$ to $10^5$, or that is, those cells existed stably in the cubes.

On the other hand, as in Fig. 2, it is known that in the rock wool cubes as inoculated with only the pathogen, the number of the pathogenic cells, which is represented by ● - ●, greatly varied with the lapse of time.

Next referred to is Fig. 3, from which it is known that the tomato plants growing on the rock wool cubes not treated with the non-pathogenic F13 (in the control group, represented by ● - ●, ) were wilted at their external parts in 105 days after the transplantation of the plants onto the cubes, and thereafter the degree of the severity of the Fusarium crown and root rot disease of the plants increased, after all resulting in that many diseased plants were dead.

On the other hand, as in Fig. 3, one of the eight tomato plants growing on the rock wool cubes as treated with the non-pathogenic F13 (in the test group, represented by ■ - ■) was wilted at its external parts in 128 days after the transplantation of the plants onto the cubes, while the others were not even in 133 days after the transplantation.

Naturally, the tomato plants growing on the rock wool cubes not inoculated with the pathogen (in the reference group, represented by ▲ - ▲) were not diseased at all.

In addition, 133 days after the transplantation, the tomato plants in those groups were estimated with respect to (1) the browning of their basal stem parts, (2) their height, and (3) the weight of the tomato fruit per bunch as yielded by them. The data obtained are shown in Table 3 and Table 4. In Table 4 the weight of the tomato fruit indicates the total weight thereof yielded by 8 tomato plants tested herein, and the data with parethesises indicate ratios relative to the yield in the control group not incoculated with the pathogen.

In Table 3, the degree of disease incidence was obtained according to the following equation.

$$\text{Degree of Disease Incidence} = [\Sigma \text{ (number of plants having the same disease index)} \times \text{(disease index)}] / \{\text{(number of all plants tested)} \times 4\}] \times 100$$

The disease index is as follows:

0: The plant was not browned at its vascular bundles and piths.
1: The plant was slightly browned at its vascular bundles.
2: The plant was seriously browned at its vascular bundles, but was not at its piths.
3: The plant was seriously browned at its vascular bundles and piths.
4: The plant was dead.

Table 3

|  | Degree of Disease Incidence in Plants at Their Basal Stems (degree of browning) |
|---|---|
| Treated with F13 Invention (test group of the invention) | 41.6 b |
| Not Treated with F13 (control group) | 87.5 a |
| Not Inoculated with Pathogen (reference group) | 1.6 c |

*a to c: These mean no significance in the same character in Duncan's New Multiple Range Test (5 %).

As in Table 3 showing the results from the observation of the tomato plants at their vascular bundles and piths in the basal stems with respect to the degree of browning of the parts, as made in 133 days after the transplantation of the plants, it is known that the plants growing on the rock wool cubes not treated with the non-pathogenic F13 (control group) were seriously browned to have a degree of disease incidence of 87.5, while those growing on the rock wool cubes as treated with the non-pathogenic F13 (test group of the invention) were, though being somewhat browned to have a degree of disease incidence of 41.6, were better than the plants growing on the cubes not treated with the non-pathogenic F13 (control group).

Table 4

| | Height | Weight of Fruit per Bunch (g) | | | | | | | Total Weight (g) |
|---|---|---|---|---|---|---|---|---|---|
| | | 1st bunch | 2nd bunch | 3rd bunch | 4th bunch | 5th bunch | 6th bunch | 7th bunch | |
| Treated with F13 (test group of the invention | 250 a | 7290 | 5020 | 3315 | 2480 | 1185 | 750 | 150 | 20190a |
| Not Treated with F13 (control group) | 210 b | 8200 | 3010 | 1480 | 755 | 320 | 45 | 0 | 13810b (70) |
| Not Inoculated with Pathogen (reference group) | 253 a | 6020 | 5490 | 3515 | 2390 | 1580 | 610 | 105 | 19710a (100) |

*a to c: These mean no significance in the same character in Duncan's New Multiple Range Test (5 %).

As in Table 4, it is known that the height of the tomato plants in the group not treated with the non-pathogenic F13 (control group) was short, while there was no difference in the height between the plants in the group treated with the non-pathogenic F13 (test group of the invention) and those in the group not inoculated with the pathogen (reference group).

Regarding the fruit weight, there was found no difference both in the weight per bunch and in the total weight between the plants in the group treated with the non-pathogenic F13 (test group of the invention) and those in the group not inoculated with the pathogen (reference group). On the other hand, in the tomato plants in the group not treated with the non-pathogenic F13 (control group), as having caught the Fusarium crown and root rot disease in tomato, the fruit weight of from the 2nd to 7th bunches was reduced, resulting in the reduction in the total fruit weight to 70 % of that as yielded by the plants in the group not inoculated with the pathogen (reference group).

Summarizing the test data as above, it is understood that the non-pathogenic F13, Fusarium oxysporum (FERM BP-5825), is highly effective against the Fusarium crown and root rot diseases in tomato plants being grown in hydroponics using rock wool culture.

Example 2:

Two-leaves-stage tomato seedlings (variety: House Momotaro) were transplanted on the rock wool cubes for growing plants as prepared in Production Example 1, and grown in a cultivation tank, through which was circulated a culture medium (containing 1.5 g/liter of Otsuka House No. 1, 0.8 g/liter of Otsuka House No. 2 and 0.2 g/liter of Otsuka House No. 8), for 40 days.

On the other hand, a liquid was prepared by incubating cells of Fusarium oxysporum f. sp. radicis-lycopersici (this causes a Fusarim crown and root rot disease in tomato plants) in a potato sucrose broth medium (PSB) with shaking at 25°C for 6 days, and mixed with soil (which is Taihei Engei Baido produced by Taihei Bussan Company ) to make the soil have the cells in an amount of $10^5$ cells/cm$^3$. The thus-prepared soil was put into 1/2000 a-Wagner pots, in each of which was transplanted the tomato seedling along with the rock wool cube on which it was growing. Three series each having five pots were prepared in each group. All pots were put in a glass greenhouse, in which the lowermost temperature was set at not lower than 10 °C.

121 days after the transplantation, the degree of disease incidence in the tomato plants at their external parts was estimated. The data obtained are shown in Table 5. The negative control group differs from the other groups in that in the former the soil used were not inoculated with the pathogen causing a Fusarium crown and root rot diseases in tomato plants, while in the latter the soil used were inoculated with the pathogen.

The degree of disease incidence was obtained according to the following equation.

Degree of Disease Incidence =[Σ (number of plants having the same disease index) × (disease index)} / {(number of all plants tested) × 4} ] × 100

The disease index is as follows:

    0: The plant was not diseased.
    1: The plant was partly wilted at its shoot apices.
    2: The plant was partly wilted at its leaves.
    3: The plant was wholly wilted.
    4: The plant was dead.

Table 5

| | Degree of Disease Incidence |
|---|---|
| Treated with F13 (test group of the invention) | 5.4 b |
| Not Treated with F13 (control group) | 45.2 a |
| Not Inoculated with Pathogen (reference group) | 0.0 b |

*a to b: These mean no significance in the same character in Duncan's New Multiple Range Test (5 %).

As in Table 5 showing the results from the observation of the tomato plants with respect to the degree of disease incidence at their external parts, as made in 121 days after the transplantation of the plants, it is known that many plants growing on the rock wool cubes not treated with the non-pathogenic F13 (control group) were dead, while most of those growing on the cubes as treated with the non-pathogenic F13 (test group of the invention) were not diseased.

In addition, 121 days after the transplantation, the tomato plants in those groups were estimated with respect to the browning of their basal stem parts. The data obtained are shown in Table 6. The degree of disease incidence was obtained according to the following equation.

Degree of Disease Incidence =[Σ (number of plants having the same disease index) × (disease index)} / {(number of all plants tested) × 4) ] × 100

The disease index is as follows:

    0: The plant was not browned at its vascular bundles and piths.
    1: The plant was slightly browned at its vascular bundles.
    2: The plant was seriously browned at its vascular bundles, but was not at its piths.
    3: The plant was seriously browned at its vascular bundles and piths.
    4: The plant was dead.

Table 6

| | Degree of Disease Incidence in Plants at Their Basal Stems (degree of browning) |
|---|---|
| Treated with F13 (test Group of the Invention) | 25.2 b |
| Not Treted with F13 (control group) | 63.8 a |
| Not Inoculated with Pathogen (reference group) | 0.0 c |

*a to c: These mean no significance in the same character in Duncan's New Multiple Range Test (5 %).

As in Table 6 showing the results from the observation of the tomato plants at their vascular bundles and piths in the basal stems with respect to the degree of browning of the parts, as made in 121 days after the transplantation of the plants, it is known that the plants growing on the rock wool cubes not treated with the non-pathogenic F13 (control group) were seriously browned to have a degree of disease incidence of 63.8, while those growing on the cubes as treated with the non-pathogenic F13 (test group of the invention) were, though being somewhat browned to have a degree of disease incidence of 25.2, were better than the plants growing on the cubes not treated with the non-pathogenic F13 (control group).

Summarizing the test data as above, it is understood that the non-pathogenic F13, Fusarium oxysporum (FERM BP-5825), is highly effective against the Fusarium crown and root rot diseases in tomato plants being grown in soil culture using rock wool.

The rock wool for growing plants of claim 1 of the invention comprises spore cells or mycelial cells of non-pathogenic microorganisms. Using this in growing plants, it is possible to prevent any infectious plant diseases to be caused by pathogens.

Since the rock wool for growing plants comprises non-pathogenic microorganisms with no synthetic fungicides, its use has no problems in its safety. The rock wool for growing plants can maintain the original, high cell population density of the cell for several weeks or longer. Therefore, this can be used in- the field of growing plants, after having been sold in the market.

In the mechanism of the rock wool for growing plants of the invention that expresses the ability to prevent plant diseases to be caused by soil-borne infectious pathogens, neither antagonism nor competition between non-pathogenic microorganisms and pathogenic microorganisms is admitted. In the rock wool, even if the parts as inoculated with non-pathogenic microorganisms are distant from those as infected with pathogenic microorganisms, the rock wool is still effective. Therefore, it is believed that the pre-inoculation of the rock wool with non-pathogenic microorganisms shall induce systemic resistance to pathogens.

As in claims 2 and 3 of the invention, where non-pathogenic microorganisms of the genus Fusarium are used, the rock wool is effective in preventing soil-borne infectious diseases, especially a Fusarium crown and root rot in tomato disease.

According to the invention of claims 4 to 6, it is possible to efficiently produce the rock wool for growing plants of claim 1 of the invention, especially that of claim 2 comprising spore cells or mycelial cells of non-pathogenic microorganisms of the genus Fusarium.

Where the rock wool for growing plants is dried to have a water content of not larger than 15 % as in claims 7 and 8, it is possible to prevent the non-pathogenic microorganisms existing in the rock wool from growing or sprouting and to prevent any harmful microorganisms that may be in the rock wool from growing. In addition, it is also possible to distribute such dry rock wool in the market. The dry rock wool may be wetted in the actual field of growing plants to make the non-pathogenic microorganisms existing therein well sprout. Using the thus-wetted rock wool, any soil-borne infectious diseases can be prevented in growing plants.

As in claims 9 and 10, the rock wool of the invention can be used in growing plants, after having been wetted. This is advantageous in that any soil-borne diseases can be prevented with no use of synthetic fungicides.

In growing plants in that manner, plants can be grown without being diseased. In that, in particular, tomato plants are effectively prevented from catching a Fusarium crown and root rot disease.

INDUSTRIAL APPLICABILITY

Accordingly, the invention is favorably utilized in the field of agriculture and horticulture.

## Claims

1. Rock wool for growing plants, which contains spore cells or mycelial cells of non-pathogenic microorganisms.

2. The rock wool for growing plants as claimed in claim 1, in which the non-pathogenic microorganisms are those of the genus <u>Fusarium</u>.

3. The rock wool for growing plants as claimed in claim 2, in which the non-pathogenic microorganisms of the genus <u>Fusarium</u> are those of <u>Fusarium Oxysporum</u> (FERM BP-5825).

4. A method for producing rock wool for growing plants of claim 1, which comprises inoculating rock wool with a suspension of spore cells or mycelial cells of non-pathogenic microorganisms, followed by drying the rock wool.

5. The method as claimed in claim 4, in which the non-pathogenic microorganisms are those of the genus <u>Fusarium</u>.

6. The method as claimed in claim 5, in which the non-pathogenic microorganisms of the genus <u>Fusarium</u> are those of <u>Fusarium oxysporum</u> (FERM BP-5825).

7. The method as claimed in claim 4, in which the rock wool is dried to have a water content of not larger than 15 %.

8. The method as claimed in claim 6, in which the rock wool is dried to have a water content of not larger than 15 %.

9. A method for growing plants using rock wool, which comprises wetting a rock wool for growing plants of claim 1, followed by growing plants on the rock wool.

10. A method for growing plants using rock wool, which comprises wetting a rock wool for growing plants of claim 2, followed by growing plants on the rock wool.

# FIG. 1

■—■ : Number of cells of F13
●—● : Number of the Pathogenic Cells

Number of Days after the Transplantation

## FIG. 2

●—● : Number of the Pathogenic Cells

y-axis: log number cfu/cm$^3$ (4, 5, 6)

x-axis: 0  13  34  48  62  76  90  104  118

Number of Days after the Transplantation

## FIG.3

■ — ■ : Treated with F13
    (test group of the invention)
● — ● : Not treated with F13(control group)
▲ — ▲ : Not Inoculated with Pathogen
    (reference group)

Degree of Disease Severity

Number of Days after the Transplantation

| INTERNATIONAL SEARCH REPORT | International application No. |
|---|---|
| | PCT/JP97/00551 |

**A. CLASSIFICATION OF SUBJECT MATTER**

Int. Cl$^6$  A01G7/00, A01G1/00, A01N63/00

According to International Patent Classification (IPC) or to both national classification and IPC

**B. FIELDS SEARCHED**

Minimum documentation searched (classification system followed by classification symbols)

Int. Cl$^6$  A01G7/00, A01G1/00, A01N63/00

Documentation searched other than minimum documentation to the extent that such documents are included in the fields searched

| | |
|---|---|
| Jitsuyo Shinan Koho | 1922 – 1997 |
| Kokai Jitsuyo Shinan Koho | 1971 – 1997 |
| Toroku Jitsuyo Shinan Koho | 1994 – 1997 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP, 7-97938, B (Asahi Chemical Industry Co.,<br>Ltd.),<br>October 25, 1995 (25. 10. 95)(Family: none) | 1, 4, 5-10 |
| Y | JP, 7-110802, B (Mitsui Toatsu Chemicals, Inc.<br>and another),<br>November 29, 1995 (29. 11. 95)(Family: none) | 2 - 10 |
| Y | JP, 6-256126, A (Japan Tobacco Inc.),<br>September 13, 1994 (13. 09. 94)(Family: none) | 2 - 10 |
| Y | JP, 6-122607, A (Shizuoka Prefecture and<br>another),<br>May 6, 1994 (06. 05. 94)(Family: none) | 2 - 10 |

☐ Further documents are listed in the continuation of Box C.       ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| May 20, 1997 (20. 05. 97) | June 3, 1997 (03. 06. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)